# EUROPEAN PATENT APPLICATION

(11) **EP 4 020 345 A1**
(43) Date of publication of application: **29.06.2022**
(21) Application number: 20306698.0
(22) Date of filing: 28.12.2020
(51) Int. Cl.: G06Q 10/04

(54) **CLOUD SERVER, NODE, SYSTEM AND METHOD FOR ASSESSING TREE AND FOREST HEALTH**

(71) Applicant: foldAI Turner und Partner Data Scientists PartG, 81369 München (DE)
(72) Inventor: Carmantini, Giovanni, 81369 München (DE); Charennat, Alicia, 68230 Turckheim (FR); Förstner, Friedrich, 68230 Turckheim (FR); Isik, Mehmet Can, 81476 München (DE); Turner, Jake, 81369 München (DE)
(74) Representative: Hoffmann Eitle

(57) **Abstract**

A cloud server, a node, a system and a method for assessing tree and forest health by measurement of tree communication through air borne particles are provided.

## Description

### Technical Field

The present invention relates to a cloud server, a node, a system and a method for assessing tree and forest health by measurement of tree communication through air borne particles.

### Technical Background

Trees emit a wide range of Biogenic Volatile Organic Compounds (BVOCs), where different processes or events elicit different emission profiles. It has been shown that such emissions function as a way for trees to exchange information between each other, so that the emission of BVOCs are akin to communication events. Example processes eliciting BVOC emissions are fruit ripening (the BVOC indicating this being methanol), tree senescence (ethylene), root anoxia (ethanol, methanol, acetaldehydes), heat stress (isoprene, monoterpenes), drought stress (isoprene, monoterpenes), herbivore attack (monoterpenes). A monitoring system able to tap into these communication events would allow for the precise tracking of a range of important quantities and factors relating to the overall health of the forest and that are otherwise elusive.

The key obstacles in capturing these events are that the emission profiles they elicit are highly dynamic, where the level of the different emitted molecules changes over time, and that communication events overlap, such that BVOC readings contain information from multiple events at the same time, which must be teased apart to characterize the single events.

Particular relevant factors acting on BVOC readings are heat stress, drought stress and herbivore attacks. These may thus be taken as example factors acting on BVOC readings and may be used as relevant factors in view of the goal of detecting herbivore attack events.

When an herbivore feeds on a tree's leaves, the tree communicates to other trees that an attack is taking place. In turn, the trees receiving this signal can respond to the attack by e.g. increasing the production of irritants, to discourage the attacker and drive it away from the area. Heat and drought stress are instead for the most part a consequence of the tree's own local climatic and soil conditions, which are in turn tied to the overall climatic and soil conditions in the forest area.

There are key differences in the emission changes caused by heat or drought stress, when compared with changes caused by herbivore attacks, e.g. timescales: heat and drought stress emission changes develop slowly over timescales of weeks and even months, whereas changes due to herbivore attacks can develop quickly in the order of minutes, then linger for days or weeks; localization: because of their nature heat and drought stress develop over large patches of forest, whereas herbivore attacks tend to be more localized; signal pattern: each of the contributions to BVOCs emissions from heat stress, drought stress and herbivore attacks cause specific patterns in the time evolution of the sensor readings, e.g. heat stress first causes an increase in emissions which then evolves in a steep drop if stress is sustained.

Because of these differences, it is possible to meaningfully pick apart the contributions of heat stress, drought stress and herbivore attacks in causing a certain BVOC emission profile. However, in order to do so, and thus robustly disambiguate herbivore attack communication events (HACE), a monitoring system must be able to integrate present and historical information on both local and global forest conditions.

Moreover, an important aspect of such systems is the power consumption at the location of the measurement. Thus, keeping the power consumption at a minimum while maintaining good monitoring would improve the performance characteristics of the entire system.

Such a monitoring system will be discussed in the following. Because the key feat of the system is a product of the orchestration between local sensing nodes and global information integration at the cloud level, the example will trace a path from sensing node to cloud and back.

### Summary

It is an object of the invention to provide for an improved performance at reduced power requirements.

This object is solved by the subject matter of the independent claims, advantageous embodiments are disclosed in the dependent claims.

### Brief description of the drawings

Embodiments of the present invention, which are presented for better understanding the inventive concepts, but which are not to be seen as limiting the invention, will now be described with reference to the figures in which:
- Fig. 1: shows a flow diagram of a method for assessing tree and forest health via measurement of tree communication through air borne particles,
- Fig. 2: shows a system comprising a cloud server and two nodes for assessing tree and forest health via measurement of tree communication through air borne particles,
- Fig. 3: shows how the overall intensity readings of a single volatile organic compound might result from a combination of factors,
- Fig. 4: shows a configuration of a server (upper) and a configuration of a node (lower),
- Fig. 5: shows further details of a node (upper) and of a sensor (lower),
- Fig. 6: shows a graph model for BVOC source identification,
- Fig. 7: shows a configuration of a system comprising a node and a cloud server,
- Fig. 8: shows a configuration of a system comprising a node and a cloud server including an update process in the node,
- Fig. 9: shows a configuration of a system comprising N nodes and a cloud server,
- Fig. 10: shows an overview of the functionality of the system,
- Fig. 11: shows two configurations of a node, and
- Fig. 12: shows an overview of the system.

### Detailed Description

According to an embodiment of the present invention, there is provided a cloud server for assessing tree and forest health by measurement of tree communication through air borne particles, the cloud server being configured to: receive first local environment data including data related to air borne particles from a plurality of nodes; transmit second local environment data to the plurality of nodes; receive at least one first prediction of the local environment from at least one of the plurality of nodes; perform a second prediction of the local environment based on the first local environment data and the second local environment data; compare the second prediction of the local environment with at least one of the first predictions of the local environment; and when the difference between the at least one of the first predictions and the second prediction is determined to exceed a threshold value, request updated first local environment data and/or transmit updated second local environment data to at least one of the plurality of nodes.

Here, data could be the type of data, that is, the parameter, the value, that is, the value of the parameter; further, data can contain a temporal behavior, that is, the data is gathered for several time instances and compared to each other; data may also comprise a distribution of the values measured.

It is noted that prediction herein describes representation of the state of the tree and/or forest and its environment. For example, this representation can be set of all measured data as well as further information such as an indication whether a HACE is taking place or is about to take place.

In more detail, an example of a prediction may be that a node 200 measures local data including BVOCs data and then performs processing on this data. This data may be a time series of data such that an analysis of the temporal behavior and change of the data is possible. If one of the measured BVOC data corresponds to monotherpene, then it is known that this data corresponds to heat stress, drought stress, and herbivore attacks. Thus, the node may perform processing on this data on the basis of the knowledge that herbivore attacks have a different temporal profile in monotherpene data. Thus, the processing may lead to the outcome that an herbivore attack is likely happening or is likely not happening. This outcome can be an example of a prediction.

Another example of a prediction may be based on the connection between monotherpene data and drought stress. That is, if there is very little rain for a prolonged time in the vicinity of a node, the node may measure this by means of a corresponding sensor. Taking this together with a model that knows about the relationship between drought and changes in the monotherpene data, the node may process the rain data and as a result of this processing may make the possible prediction that due the prolonged lack of rain a change in the monotherpene data is to be expected.

According to an embodiment of the present invention, there is provided a node for assessing tree and forest health via measurement of tree communication through air borne particles, the node being provided in the vicinity of a tree and being configured to: sense first local environment data including data related to air borne particles; receive second local environment data from a cloud server; perform a first prediction of the local environment based on the first local environment data and the second local environment data; transmit the first local environment data to the cloud server; and transmit the first prediction of the local environment to the cloud server.

According to an embodiment of the present invention, there is provided a system for assessing tree and forest health via measurement of tree communication through air borne particles, the system comprising at least one node according to an embodiment of the present invention and a cloud server according to an embodiment of the present invention.

According to an embodiment of the present invention, there is provided a method for assessing tree and forest health by measurement of tree communication through air borne particles, the method comprising the steps of: receiving first local environment data including data related to air borne particles from a plurality of nodes; transmitting second local environment data to the plurality of nodes; receiving at least one first prediction of the local environment from at least one of the plurality of nodes; performing a second prediction of the local environment based on the first local environment data and the second local environment data; comparing the second prediction of the local environment with at least one of the first predictions of the local environment; and when the difference between the at least one of the first predictions and the second prediction is determined to exceed a threshold, requesting updated first local environment data and/or transmitting updated second local environment data to at least one of the plurality of nodes.

According to an embodiment of the present invention, there is provided a method for assessing tree and forest health via measurement of tree communication through air borne particles, the method comprising the steps of: sensing first local environment data including data related to air borne particles; receiving second local environment data from a cloud server; performing a first prediction of the local environment based on the first local environment data and the second local environment data; transmitting the first local environment data to the cloud server; and transmitting the first prediction of the local environment to the cloud server.

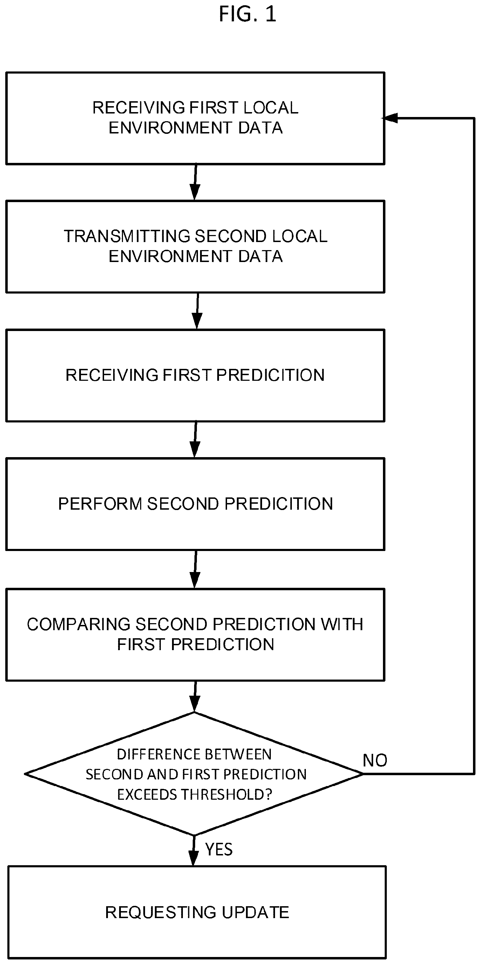

Fig. 1 shows a flow diagram of a method for assessing tree and forest health via measurement of tree communication through air borne particles according to another embodiment of the invention. This method may be performed by a cloud server 100; further, this method may be performed via interaction with a plurality of nodes 200.

In a first step, first local environment data including data related to air borne particles from a plurality of nodes 200 are received. In a next step, second local environment data to the plurality of nodes 200 are transmitted. It is noted that the order of these two steps is not constrained, that is, the step of transmitting second local environment data may be performed before the step of receiving first local environment data.

In a further step, at least one first prediction of the local environment from at least one of the plurality of nodes is received. Further, a second prediction of the local environment based on the first local environment data and the second local environment data is performed. It is noted that also these two steps are not constrained in their order, that is, the step of performing the second prediction can be done before the step of receiving the first prediction.

In a further step, the second prediction of the local environment is compared with at least one of the first predictions of the local environment. Herein, it is determined whether the difference between the at least one of the first predictions and the second prediction exceeds a threshold. If this is the case, that is, the threshold is exceeded (YES in Fig. 1), an update is performed. This update may comprise requesting updated first local environment data and/or transmitting updated second local environment data to at least one of the plurality of nodes. If the threshold is not exceeded (NO in Fig. 1), the method may return to the beginning and may continue the process iteratively.

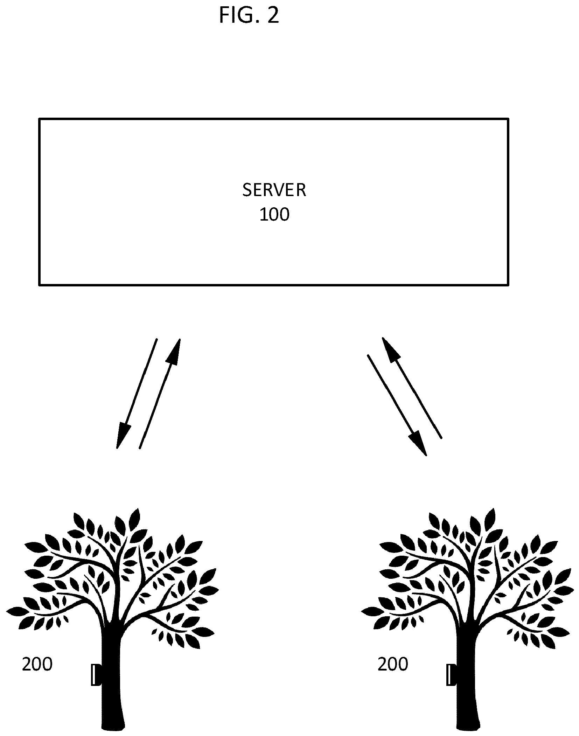

Fig. 2 shows a system comprising a cloud server 100 and two nodes 200 for assessing tree and forest health via measurement of tree communication through air borne particles.

As illustrated, the cloud server 100 and each of the nodes 200 are able to exchange data bilaterally between each other. Even tough not depicted, exchange of data between the nodes 200 may be possible as well.

The cloud server 100 may be able to perform any of the methods for assessing tree and forest health via measurement of tree communication through air borne particles. In particular, the cloud server 100 may be a cloud server 100 for assessing tree and forest health by measurement of tree communication through air borne particles, the cloud server 100 being configured to: receive first local environment data including data related to air borne particles from a plurality of nodes 200; transmit second local environment data to the plurality of nodes 200; receive at least one first prediction of the local environment from at least one of the plurality of nodes 200; perform a second prediction of the local environment based on the first local environment data and the second local environment data; compare the second prediction of the local environment with at least one of the first predictions of the local environment; and when the difference between the at least one of the first predictions and the second prediction is determined to exceed a threshold value, request updated first local environment data and/or transmit updated second local environment data to at least one of the plurality of nodes 200.

It is noted that the updated data does not only comprise that data is updated by newly measured data, that is, the same type of data is measured another time, but also comprises that the data itself is updated. That includes, for example, that the configuration of the nodes and sensors in the nodes can be changed. Equally, the protocol used in the nodes, the models used in the nodes to model e.g. the environment, and the parameters measured can be updated. In other words, updating is not restricted to replacing old data with newer data but also includes changing the way of operation related to obtaining the data.

It is noted that the first local environment data may be related to at least one of a Biogenic Volatile Organic Compound, the body of a tree, temperature, humidity, pressure, light, and sound. Light may in particular include the light visible to the human eye, that is light with a wavelength of roughly 380 to 750 nm.

That is, the first local environment data may be data that can be measured by sensors provided by the node. Biogenic Volatile Organic Compounds relate to airborne particles output by trees. Analyzing the various different types of these, the temporal changes of them separately as well as in combination gives insight into the state of the tree, its environment as well as its conditions, e.g. its health. Data related to the body of the tree may indicate characteristics such a growth or equally decline of the tree, change of the same, constitution of the tree, and the like. In other words, also the data related to the body of the tree may contain information related to the condition of the tree and may give insight into its health. Further, also well-known parameters such as temperature, humidity, pressure, light, and sound can be part of the first local environment data measured by the node, i.e. sensors of the node. In other words, temperature, humidity, pressure, light, and sound in the vicinity of the node can be measured in order to gather first local environment data.

Further, it is noted that the second local environment data may be related to at least one of satellite data, data from an aircraft, ground based sensor data, manual measurements, weather data, and soil data. In this, satellite data may be at least one of RGB images, infrared images, while soil data may be at least one of humidity, temperature, fungi, nitrogen and phosphate concentration, pH, humus, and salinity.

The first and the second local environment data are not mutually exclusive. For example, it is clear that weather data and soil data are closely related to humidity and temperature data, that ground based sensor data is generally in agreement with any data measured by the node. From this it can be understood that in some embodiments of the present invention the second local environment data includes the first local environment data. Further, in some embodiments of the present invention, the second local environment data includes data that is not part of the first local environment data. For example, satellite data such as satellite image data is a type of data that a typical sensor included in a node provided in the vicinity of a tree may not provide. This satellite data may be meteorological information about the area in which the node is provided and may thus contribute to the other data that may be part of the first and the second local environment data.

The first local environment data may be values for a first set of parameters and the second local environment data may be values for a second set of parameters. The parameters may correspond to for example temperature, humidity, BVOCs, weather, light or light conditions, and rain. For example, the parameters corresponding to the first local environment data may be temperature, humidity and BVOCs while the parameters corresponding to the second local environment data may be weather, light or light conditions, and rain. The first and second sets of parameters may be corresponding to one another, e.g. including local temperature measured by a sensor and by satellite or may differ from one another or may overlap.

Moreover, the cloud server 100 may be able to determine that update is to be performed on the basis of comparing the various predictions. In detail, the cloud server 100 may further be configured to: compare the second prediction of the local environment with the at least one of the first predictions of the local environment and the result thereof with at least one predetermined threshold value, determine, based on a result of the comparison, which part of the first local environment data and which part of the second local environment data is required to sync the at least one of the first predictions of the local environment and the second predictions of the local environment with each other, wherein further the requested updated first local environment data and/or the transmitted updated second local environment data corresponds to this determined required data.

In other words, the cloud server 100 compares the first and the second prediction and is able to determine on the basis of this comparison on which side of the system, node 100 and/or cloud server 100, an update has to be performed.

In more details, a prediction can be represented by a set of numbers that can be arranged in a vector, a matrix or a tensor. Thus, a difference between two predictions, both represented in the same format, can be calculated. This difference is again a set of numbers arranged in a vector, a matrix or a tensor This difference can then be compared to a predetermined threshold value or a plurality of predetermined threshold values. This comparison may take different forms. For example, a single value of this difference may be considered and compared to a threshold value. In another example, an overall value of all values of the difference may be calculated. Further, also a subset of all values of the values of the difference may be considered. In all of these cases, the different values may be weighted differently. This allows to weight different aspects of the predictions and the difference therebetween. A comparison based on a single value may be useful to determine that a certain single aspect of a prediction is drifting gravely apart between the first and the second prediction. This may help to identify a single cause for this divergence without modifying the rest. An overall comparison taking into account all values may indicate that the predictions generally disagree substantially and thus a more substantial update has to be performed.

Further, the step of comparing the second prediction of the local environment, the at least one of the first predictions of the local environment, and the at least one predetermined threshold value may further comprise: a predetermined threshold value that indicates that first local environment data is required to sync the at least one of the first predictions of the local environment and the second predictions of the local environment with each other, or that second local environment data is required to sync the at least one of the first predictions of the local environment and the second predictions of the local environment with each other.

In line with previous examples of the predictions, the following may be an example of a comparison: Based on satellite data and weather data being part of the second local environment data, the second prediction may be that there is very likely an herbivore attack happening. This might be for example due to the fact that the second local environment data corresponds to data that has been previously established to be correlated to an herbivore attack. Thus, the second prediction is that a herbivore attack is happening. However, the first local environment data, in this example including the monotherpene data, observes no fluctuations indicating any herbivore attack. Thus, the first prediction is that no herbivore attack is happening. In this simple example, the prediction is merely this quantity and thus the only value to be compared. A Boolean representation of "HACE happening" and "HACE not happening" may be appropriate. In other examples the prediction contains also other elements, for example the temperature, the humidity in the vicinity of the node and the like as discussed above; then a more complex representation may be appropriate and more complex statements are possible.

Turning back to the simple example, the comparison will determine that the predictions do not agree and since there is only one parameter with only two possible configurations, will also determine that an update is required. It is noted that in this example there is no further information to be used for determining how the update shall be performed.

In an example, based on the simple example described above, the predictions contain more than the Boolean value related to the HACE. In this case, the predictions and in turn the comparison contain more information. In such a case, the comparison might lead to the result that the first prediction is "no HACE" on the basis of monotherpene data while the second prediction is "HACE" on the basis of weather and satellite data alone. In this case, the further data describing the basis for the prediction can be considered. For example, based on the type of data used for making the prediction a value indicating the quality of the prediction can be calculated. Since such a quality can be determined for both predictions, a difference between the two values can indicate which prediction's quality is higher and thus that the data used for the other prediction requires updating.

The present invention recognizes that the overall goal is to balance accuracy of detection of HACEs and abiding to power constraints, e.g. keep power consumption at a minimum. This is a trade-off: the fewer data is collected by the node, and exchanged between cloud and node, the more the uncertainty increases in both models, that is, the model of the environment used by the cloud resp. the node, leading to a loss of performance in detection. However, this trade-off relation is not linear, because of the presence of strong space and time correlations in the data collected by nodes in the deployment, which create redundancy in information.

Thus, it is one aim of the present disclosure to provide a solution in this trade-off scenario that achieves both. In detail, the present disclosure discusses a system comprising a cloud server and a plurality of nodes wherein the system has access to these correlations, thanks both to information from the sensing nodes, and external information available on the cloud and the system can harness the redundancy by orchestrating a reduction in the amount of data which needs collecting and communicating, without causing a proportional increase in the uncertainty of its predictions or, in other words, a decrease in its predictive capabilities.

In another embodiment, the first and the second prediction may be based on the same type of data, which however is clearly obtained in different ways: The first prediction is based on the first local environment data of the node 200, thus is data mostly gathered by the node 200 itself. Different from that the second prediction is based on the second local environment data of the cloud server 100, thus is data mostly gathered by the cloud server 100 from external entities. As a result of the different sources for the data, also the corresponding predictions may deviate. Thus, by comparing not only the prediction in terms of a singular element but in more detail also comparing how the prediction is made, it can be determined that one source is more accurate than the other. For example, the data used by the cloud server 100 may be not accurate enough. Or, for example, the measurement frequency of the sensor in the node 200 may be to small. Based on such aspects, the comparison may be able to determine which data requires updating.

Further, updating the data requires resources which oftentimes want to be minimized as the system may have certain power constraints in view of the field of application, e.g. limited battery capacity.

Thus, the cloud server 100 may perform the determination which data requires updating such that the data transmitted from the cloud server 100 to the at least one node 200 is minimized.

Alternatively, the cloud server 100 may perform the determination which data requires updating such that the data requested by the cloud server 100 from the at least one node 200 is minimized.

In a very simple embodiment, these minimizations can be realized by simply blocking any update of the cloud server 100 resp. the node 200. In a slightly more elaborate example, only those updates are allowed that do not increase the amount of data send by the entity whose transferring activities shall be minimized. Implementation of the former can be realized via a further step in the above described process by checking in a last step whether the update is on the side of the node 200 (resp. the cloud server 100) and rejecting the update if the transferring activities of the node 200 (resp. the cloud server 100) shall be minimized . The latter can be implemented similarly by, instead of simply rejecting an update request, verifying that the update request does not lead to more data being sent.

Clearly, such simple implementations can be replaced by more complex logic if the number of possible updates achieving the objective is bigger. For example, there are scenarios conceivable in which the update has to be performed on the side of the node 200. However, at the same time data sent by the node 200 shall be minimized. If in such a case several solutions are possible, e.g. measuring BVOCs data more often, measuring humidity data more often or measuring light data more often, the choice can be made such that the minimization criterion is met: Among all possible solutions, the solution with the smallest increase in data sent by the node 200 (or the highest decrease in data sent by the node 200) is chosen.

As an example, also these determinations may be based on predetermined threshold values, i.e. one might define threshold values that consider these power constraints. As simple constraint could be to limit the frequency of data being sent to be less than a threshold; in this way, also the power used is constrained.

As a practical example, the result of the determination may be that the predictions have drifted apart and that the cause of this is that a part of the data used by the node 200 and provided by the cloud server 100 is outdated. The data may for example be provided to the cloud server 100 from an external source and may relate to temperature and humidity as well as soil aspects. However, as this data is static, that is, is not reacting to the actual circumstances in the vicinity of the tree monitored by the node 200, this divergence between static, external data and measured data may lead to the predictions drifting apart. Then, an appropriate response to this could be to update this data on the side of the node 200 by sending updated data from the cloud server 100 to the node 200. In this way, the amount of data sent by the node 200 can be minimized and the drift recognized between the predictions can be removed and the predictions can be synced with each other again.

Thus, in some aspects of the present invention, not only can a trade-off between monitoring and power consumption be achieved but also the power consumption of parts of the system, i.e. the node or the cloud server, can be controlled. Thus, these systems not only provide a reliable way of monitoring the health of trees while keeping power consumption at low levels but also allow to control the power consumption of the system as a whole, or in more detail, its constituents, the nodes 200 and the cloud server 100.

Further, it is noted that the cloud server 100 of an aspect of the present invention may further be configured to determine an herbivore attack communication event on the basis of the second prediction of the local environment.

Returning to the lower half of Fig. 2, the node 200, which is a node 200 for assessing tree and forest health via measurement of tree communication through air borne particles, may be configured to sense first local environment data including data related to air borne particles; receive second local environment data from a cloud server 100; perform a first prediction of the local environment based on the first local environment data and the second local environment data; transmit the first local environment data to the cloud server 100; and transmit the first prediction of the local environment to the cloud server 100.

In other words, a method for assessing tree and forest health via measurement of tree communication through air borne particles is described, the method comprising the steps of: sensing first local environment data including data related to air borne particles; receiving second local environment data from a cloud server 100; performing a first prediction of the local environment based on the first local environment data and the second local environment data; transmitting the first local environment data to the cloud server 100; and transmitting the first prediction of the local environment to the cloud server 100.

Overall, Fig. 2 thus describes a system for assessing tree and forest health via measurement of tree communication through air borne particles, the system comprising at least one node 200 as for example described above and a cloud server 100 as for example described above.

In a further example of a concrete implementation, the first local environment data may be a temporal behavior of BVOCs data, for example of monotherpene data. This may include the values of the monotherpene data as a function of time as well as the frequency of the measurement. The second local environment data may be local weather data based on satellite data. This data may also comprise the values of the respective parameters as well as the frequencies of the data points. Based on the respective local environment data, the presence of a HACE may be predicted by the respective first and second prediction. The comparison of the prediction may be based on the prediction output as well as the underlying data. In particular, a threshold for the comparison may be whether the outcomes of the predictions agree. That is, if the two predictions agree, the threshold is not exceeded, if they do not agree, the threshold is exceeded. A further threshold of the comparison may evaluate the frequency of the local environment data, that is, there might be another threshold evaluating the outcome of the first prediction on the basis of the frequency of the data used for the first prediction. A further threshold might evaluate the outcome of the second prediction on the basis of the frequency of the data used for the second prediction. These two thresholds are both defined by a, possible separate, predetermined value defining a minimum value representing a quality of the prediction based on the frequency of the data. That means that if one of these thresholds is exceeded, the corresponding data requires updating. In this case, the corresponding update may be to increase the corresponding frequency of first resp. second local environment data.

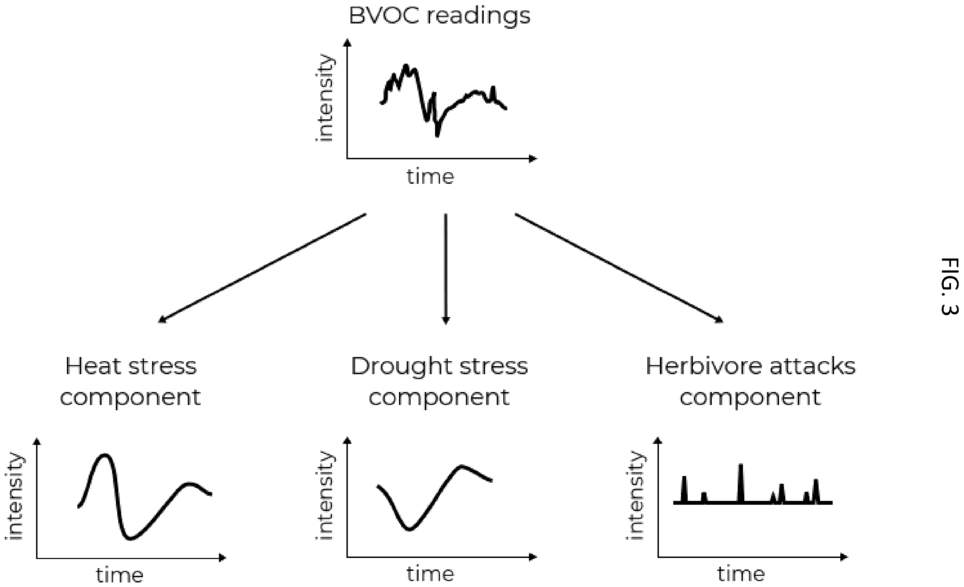

Fig. 3 shows how the overall intensity readings of a single Biogenic Volatile Organic Compound (BVOC) might result from a combination of factors. It is noted that BVOCs are compounds emitted by trees in the form of airborne particles. In other words, airborne particles may contain BVOCs of different types. In more details, it can be seen from Fig. 3 that BVOC readings, presented as an intensity as a function of time, can be split in at least three components of different meaning, category, and cause. A first component, shown on the left, is a heat stress component. This component shows a reaction to heat stress, that is, to substantial changes in temperatures. It is noted that a similar component may also be observed for negative changes in temperatures, namely cold stress in case temperatures drop substantially. In a further embodiment, negative temperature changes are to be understood as comprised in heat stress. A second component, shown in the middle, is a drought stress component. This component shows a reaction to drought stress, that is, to the lack of water or humidity in general. In a further embodiment, an extreme amount of water or humidity, represent the opposite type of stress, is to be understood as comprised in drought stress as well. A third component, shown on the right, shows an herbivore attacks component. This component shows a reaction to herbivore attacks. Note that in this example the profile of the heat stress component and the drought stress component show a slower change in time compared to the herbivore attacks component. This may be a characteristic that is useful for identifying the latter.

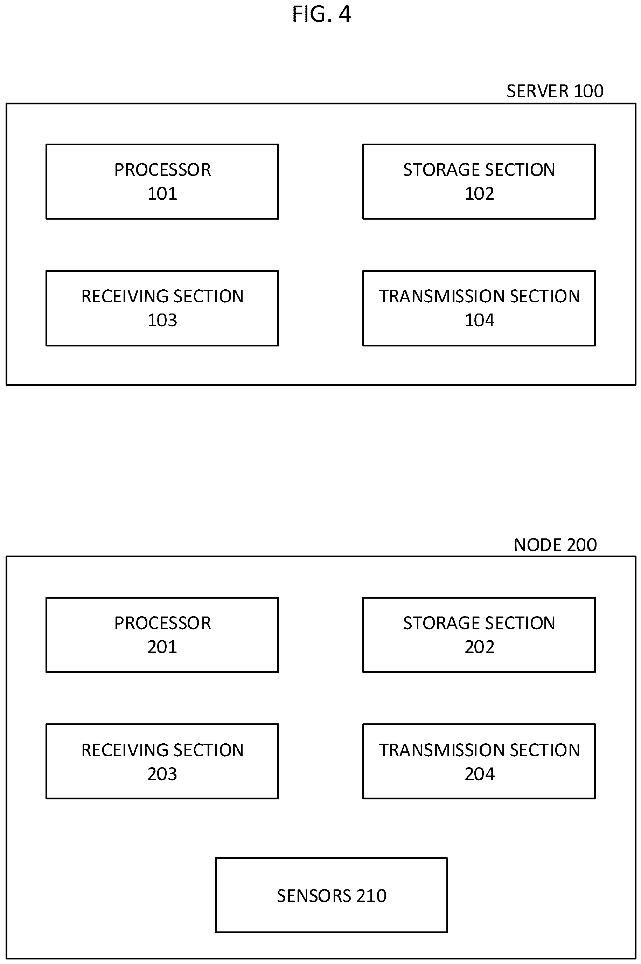

Fig. 4 shows a configuration of a cloud server 100 in the upper half of the figure and a configuration of a node 200 in the lower half of the figure.

The cloud server 100, also simply server 100 or cloud 100, comprises a processor 101, a storage section 102, a receiving section 103, and a transmission section 104.

The processor 101 may be configured to perform all steps related to calculation tasks performed by the server 100, e.g. steps related to perform predictions on the basis of the data and determining whether the predictions exceed a threshold.

The storage section 102 may be configured to store all the data, in particular the first local environment data and the second local environment data as well as the data representing the predictions, that is, the at least one first prediction of the local environment from at least one of the plurality of nodes and the second prediction of the local environment based on the first local environment data and the second local environment data.

The receiving section 103 may be configured to receive data from the plurality of nodes 200. Similarly, the transmission section 104 may be configured to transmit data to the plurality of nodes 200.

The node 200 comprises a processor 201, a storage section 202, a receiving section 203, a transmission section 204, and a plurality of sensors 210.

The processor 201 may be configured to perform all steps related to calculation tasks performed by the node 200, e.g. steps related to perform predictions on the basis of the data.

The storage section 202 may be configured to store all the data, in particular the second local environment data as well as the data representing the prediction.

The receiving section 203 may be configured to receive data from the cloud server 100. Similarly, the transmission section 204 may be configured to transmit data to the cloud server 100.

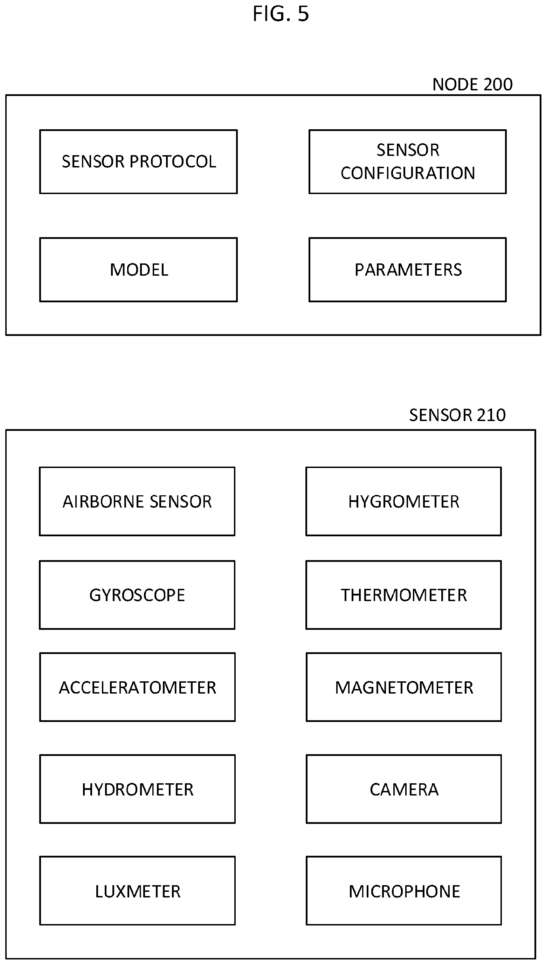

Fig. 5 shows further details of a node 200 in the upper half of the figure and further details of a sensor in 210 the lower half of the figure.

Relevant aspects of a node 200 are the sensor protocol, the sensor configuration, the model used by the sensors 210, and the parameters used by the node 200. In more detail, these aspects describe the functionality of node 200 in that they define the measurements done by the node 200. This includes among others the type of data that is measured, the frequency with which it is measured, how the node's physical configuration is adjusted in view thereof and which underlying model the node 200 uses, which influences not only which data is measured but also how the measured data influences the resulting prediction.

In connection thereto, it is noted that the cloud server 100 may be configured such that the step of requesting updated first local environment data comprises updating a local sensor protocol or a local sensor configuration.

Further, the cloud server 100 may be configured such that the step of requesting updated first local environment data comprises updating a local model.

A further possibility is that cloud server 100 may be configured such that the step of requesting updated first local environment data comprises updating local parameters.

The lower part of Fig. 5 shows possible examples of sensors used in a node 200: an airborne sensor, a hygrometer, a gyroscope, a thermometer, an accelerometer, a magnetometer, a hydrometer, a camera, a lux meter, and a microphone are typical example of sensors 210 that may be realized in the node 200. It is noted that the node 200 is not restricted to these sensors 210, that is, may include also other sensors, and may include all of these sensors or only one of these sensors or a combination of some of these sensors.

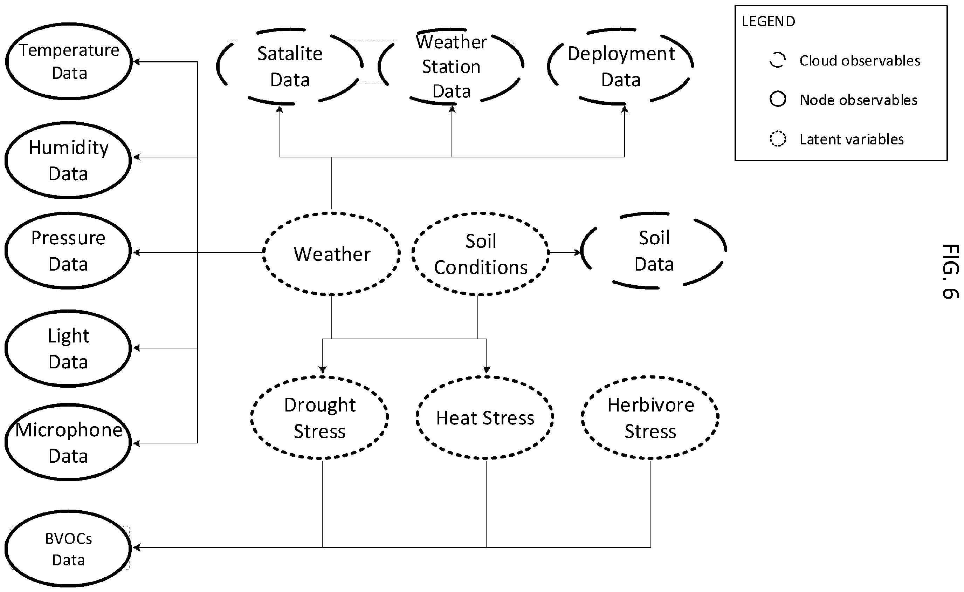

Fig. 6 shows a graph model for BVOC source identification. Therein, cloud observables, node observables, and latent variables are distinctly represented. Cloud observables relate to the cloud server 100, while node observables relate to the node 200. Typical cloud observables are satellite data, weather station data, deployment data, and soil data. Typical node observables are temperature data, humidity data, pressure data, light data, microphone data, and BVOCs data. Typical latent variables may be weather, soil conditions, drought stress, heat stress and herbivore stress. Further, Fig. 6 illustrates how these different observables and variables may relate to each other.

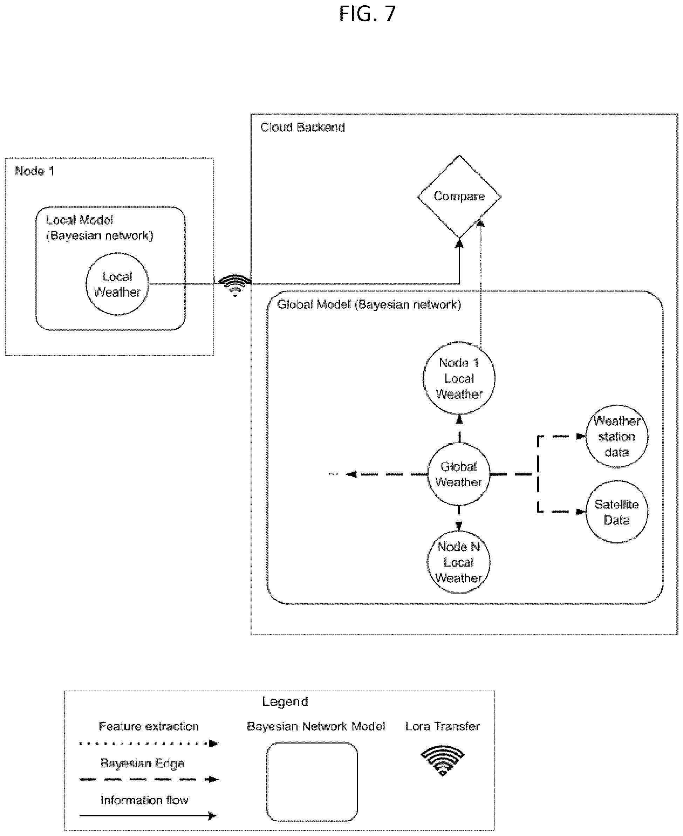

Fig. 7 shows a configuration of a system comprising a node 200 and a cloud server 100. In this example, the nodes 200 are denoted as "Node 1" to "Node N" to allow a further distinction of the different nodes 200. The example comprises a Node 1 in which a local model using a Bayesian network is used. Via long range transfer protocols (such as Lora or 5G NBIoT) data is exchanged with the Cloud Backend, which is the representation of the cloud server 100 in this example. The Cloud Backend uses a global model which also uses a Bayesian network. In this case, a global weather aspect is inferred from weather station satellite data and the like. From this, the local weather of the nodes 200, i.e. Node 1 to Node N, can be model by the global model as well. The two modelizations of the local weather of Node 1, from the local model of Node 1 and the global model of the Cloud Backend are then compared as illustrated in the upper part of the Cloud Backend.

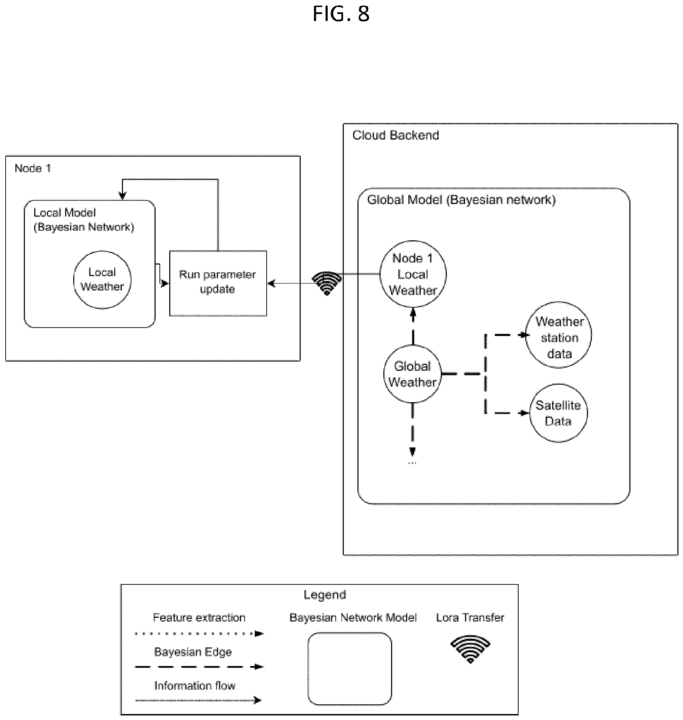

Fig. 8 shows a configuration of a system comprising a node 200 and a cloud server 100 including an update process in the node. The configuration shown in Fig. 8 is similar to the configuration shown in Fig. 7 and thus aspects common among these figures are not repeated. Fig. 8 highlights the update aspect on the side of the Node 1. By means of long range transfer protocols, the local weather of Node 1 as modelled by the global model of the Cloud Backend is transferred to Node 1, more specifically to the run parameter update module. Also the local weather as modelled by the local model of Node 1 is input in the run parameter update module. Based on these inputs, the run parameter update module updates the local model of Node 1.

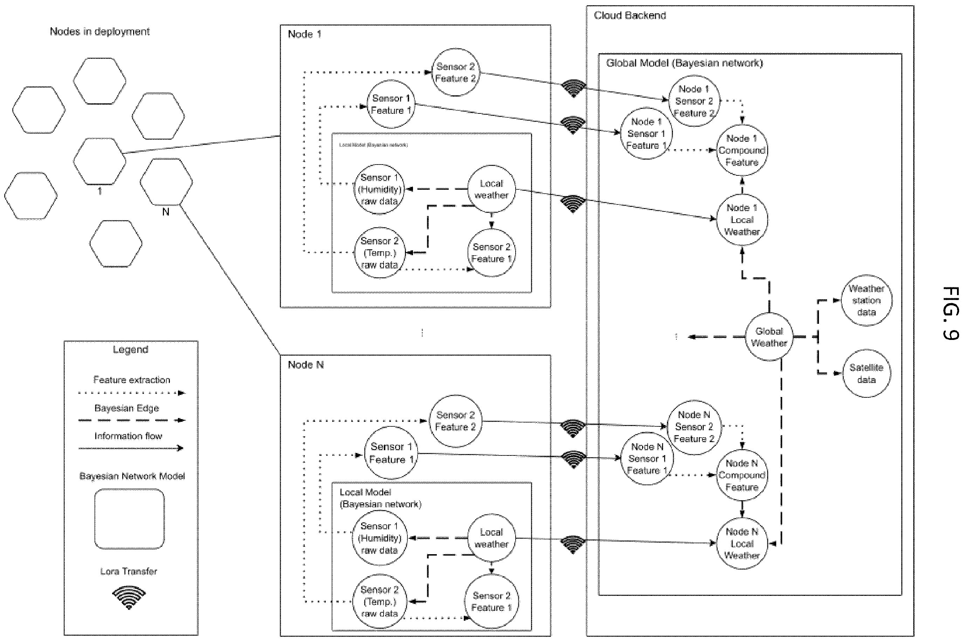

Fig. 9 shows a configuration of a system comprising N nodes and a cloud server. Fig. 9 relates to a similar system as Fig. 7 and 8. Thus, aspects common with these figures are not repeated. Differently from the previous figures, Fig. 9 contains more than one node 200, i.e. contains N Nodes, wherein only the first, Node 1, and the last, Node N, are explicitly shown. Further, the details of the weather model shown in the global model of the Cloud Backend and in the local model of the various nodes is shown in more detail. Lastly, also the nodes in deployment are sketched in Fig. 9.

More in detail, the local network of Node 1 is now depicted to include a Sensor 1 for raw data related to humidity and a Sensor 2 for raw data related to temperature. Sensor 1 provides a feature denoted Sensor 1 Feature 1, Sensor 2 provides a feature denoted Sensor 2 Feature 1 which is part of the local model and a feature denoted Sensor 2 Feature 2. Sensor 1 Feature 1 and Sensor 2 Feature 2 are transmitted via Lora transfer to the global model of the Cloud Backend as Node 1 Sensor 1 Feature 1 and Node 1 Sensor 2 Feature 2, leading to Node 1 Compound Feature, which in turn relates to Node 1 Local Weather. This further illustrates how the various data measured on the side of the nodes 200 are included in the modelization. It is noted that the same is depicted for Node N, that is, the depiction of Node 1 and Node N are the same in this example. This is, however, not a constraint. In fact, the update performed on the node as described elsewhere in this document may be common for all nodes or may be individual for each node leading to a different overall picture compared to the picture shown in Fig. 9.

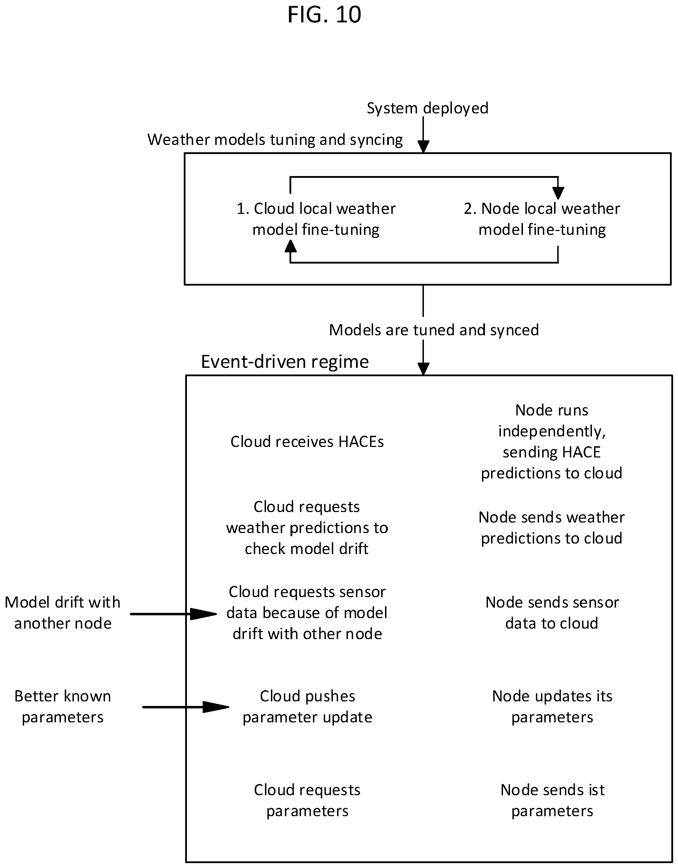

Fig. 10 shows an overview of the functionality of the system. First, the system comprising the cloud server 100 and the plurality of nodes 200 is deployed, that is, the nodes are provided in the vicinity of trees whose health is to be assessed. Next, weather models are tuned and synced. That is, in a first step the local weather model of the cloud server is fine-tuned and in a second step the local weather model is fine-tuned in response thereto. This step leads to another iteration of fine-tuning the local weather model of the cloud server. This iterative process is repeated until the models agree with respect to each other. This test of agreement can for example be realized by comparing the model by means of a difference and judging whether the resulting difference is smaller than some predetermined threshold. Once it is determined that the models are tuned and sync, the monitoring process can be started. This part is also named "event-driven regime". In this regime, given a certain event, a predetermined response is performed. For example, as depicted, if the cloud server receives HACEs, the node continues to run independently while sending HACE predictions to the cloud server. Further, if the cloud server requests weather prediction to check the model drift, the node sends weather predictions to the cloud server. If a model drift with another node 200 is determined, the cloud may request sensor data due to this model drift, thus, the node sends sensor data to the cloud server. Further, if the cloud server is provided with better known parameters, the cloud server pushes the parameter update to the node and in response the node updates its parameters. Lastly, the cloud server may request parameters and as a response the node sends these parameters.

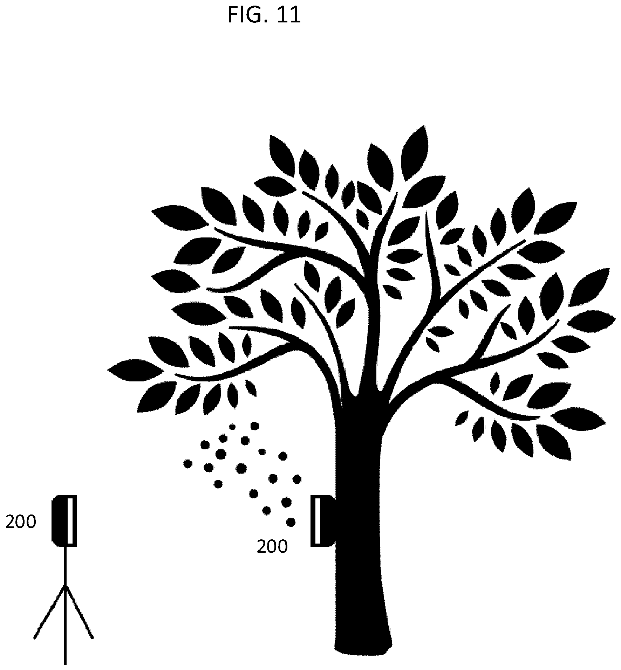

Fig. 11 shows two configurations of a node 200. The node 200 may either be attached to the tree that is to be observed as shown in the center-right part of the figure or the node 200 may be positioned separately from the tree to be observed as shown in the left part of the figure. The only requirement in both cases is that the node is provided in the vicinity of the tree such that the sensors can obtain the data relevant to the tree. Particular, access to the air borne particles is required.

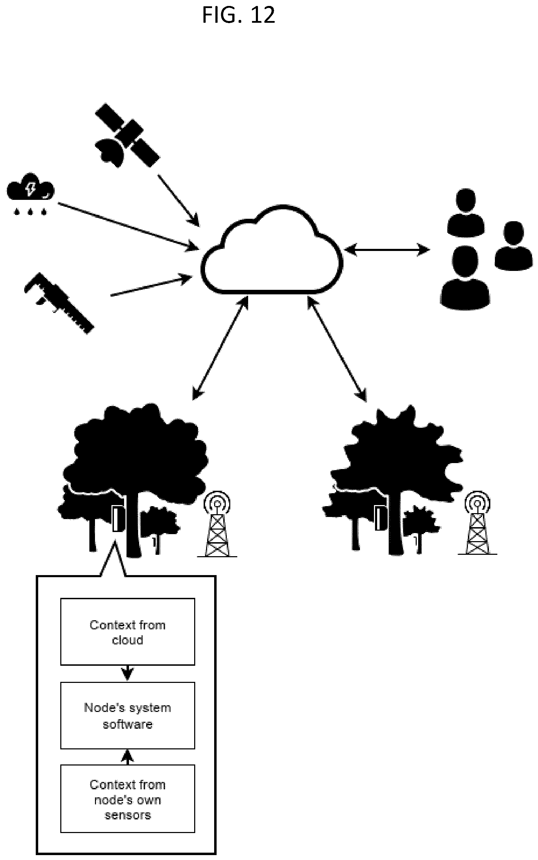

Fig. 12 shows an overview of a system. The system depicted here gives an overview of a possible big scale application of the proposed methods and devices. As described above, a node receives information, including context information, i.e. information that go beyond the information the node can obtain on its own, uses this context from the cloud server as input for its own system software. Further, the context from the node's own sensors is also used as input for the node's system software. Based on this input, the node operates. As shown in Fig. 12, there may be provided a intermediate network station between the plurality of nodes 200 and the cloud server 100 and there might be more than one such intermediate network station. Moreover, the cloud server 100 exchanges data between the plurality of nodes 200 as well as with the operators that may input data as well as receive output data form the predictions made by cloud server. Further as described also elsewhere in this document, the cloud server 100 may receive input data due to external measurements, weather, and satellite data.

## Claims

1. A cloud server (100) for assessing tree and forest health by measurement of tree communication through air borne particles, the cloud server (100) being configured to:
receive first local environment data including data related to air borne particles from a plurality of nodes (200) ;
transmit second local environment data to the plurality of nodes (200);
receive at least one first prediction of the local environment from at least one of the plurality of nodes (200) ;
perform a second prediction of the local environment based on the first local environment data and the second local environment data;
compare the second prediction of the local environment with at least one of the first predictions of the local environment; and
when the difference between the at least one of the first predictions and the second prediction is determined to exceed a threshold value, request updated first local environment data and/or transmitting updated second local environment data to at least one of the plurality of nodes (200).

2. The cloud server (100) according to claim 1, wherein the first local environment data is related to at least one of a Biogenic Volatile Organic Compound, the body of a tree, temperature, humidity, pressure, light, and sound.

3. The cloud server (100) according to claim 1 or 2, wherein the second local environment data is related to at least one of satellite data, data from an aircraft, ground based sensor data, manual measurements, weather data, and soil data.

4. The cloud server (100) according to any one of claims 1 to 3, wherein the cloud server (100) is further configured to:
compare the second prediction of the local environment with the at least one of the first predictions of the local environment and the result thereof with at least one predetermined threshold value,
determine, based on a result of the comparison, which part of the first local environment data and which part of the second local environment data is required to sync the at least one of the first predictions of the local environment and the second predictions of the local environment with each other,
wherein further the requested updated first local environment data and/or the transmitted updated second local environment data corresponds to this determined required data.

5. The cloud server (100) according to claim 4, wherein comparing the second prediction of the local environment, the at least one of the first predictions of the local environment, and the at least one predetermined threshold value comprises:
a predetermined threshold value that indicates that first local environment data is required to sync the at least one of the first predictions of the local environment and the second predictions of the local environment with each other, or that second local environment data is required to sync the at least one of the first predictions of the local environment and the second predictions of the local environment with each other.

6. The cloud server (100) according to claim 4 or 5, wherein the determination which data requires updating is done such that the data transmitted from the cloud server (100) to the at least one node (200) is minimized.

7. The cloud server (100) according to claim 4 or 5, wherein the determination which data requires updating is done such that the data requested by the cloud server (100) from the at least one node (200) is minimized.

8. The cloud server (100) according to any one of claims 1 to 7, wherein requesting updated first local environment data comprises updating a local sensor protocol or a local sensor configuration.

9. The cloud server (100) according to any one of claims 1 to 8, wherein requesting updated first local environment data comprises updating a local model.

10. The cloud server (100) according to any one of claims 1 to 9, wherein requesting updated first local environment data comprises updating local parameters.

11. The cloud server (100) according to any one of claims 1 to 10, wherein the cloud server is further configured to determine an herbivore attack communication event on the basis of the second prediction of the local environment.

12. A node (200) for assessing tree and forest health via measurement of tree communication through air borne particles, the node being provided in the vicinity of a tree and being configured to:
sense first local environment data including data related to air borne particles;
receive second local environment data from a cloud server (100);
perform a first prediction of the local environment based on the first local environment data and the second local environment data;
transmit the first local environment data to the cloud server (100); and
transmit the first prediction of the local environment to the cloud server (100).

13. A system for assessing tree and forest health via measurement of tree communication through air borne particles, the system comprising at least one node (200) according to claim 12 and a cloud server (100) according to any of claims 1 to 11.

14. A method for assessing tree and forest health by measurement of tree communication through air borne particles, the method comprising the steps of:
receiving first local environment data including data related to air borne particles from a plurality of nodes (200) ;
transmitting second local environment data to the plurality of nodes (200);
receiving at least one first prediction of the local environment from at least one of the plurality of nodes (200) ;
performing a second prediction of the local environment based on the first local environment data and the second local environment data;
comparing the second prediction of the local environment with at least one of the first predictions of the local environment; and
when the difference between the at least one of the first predictions and the second prediction is determined to exceed a threshold, requesting updated first local environment data and/or transmitting updated second local environment data to at least one of the plurality of nodes (200).

15. A method for assessing tree and forest health via measurement of tree communication through air borne particles, the method comprising the steps of:
sensing first local environment data including data related to air borne particles;
receiving second local environment data from a cloud server (100);
performing a first prediction of the local environment based on the first local environment data and the second local environment data;
transmitting the first local environmeant data to the cloud server (100); and
transmitting the first prediction of the local environment to the cloud server (100).

## Amended claims

### Amended claims in accordance with Rule 137(2) EPC.

1. A cloud server (100) for assessing tree and forest health by measurement of tree communication through air borne particles, the cloud server (100) being configured to:
receive first local environment data including data related to air borne particles from a plurality of nodes (200);
transmit second local environment data to the plurality of nodes (200);
receive at least one first prediction of the local environment from at least one of the plurality of nodes (200) using a local model using a Bayesian network;
perform a second prediction of the local environment based on the first local environment data and the second local environment data using a global model using a Bayesian network;
compare the second prediction of the local environment with at least one of the first predictions of the local environment; and
when the difference between the at least one of the first predictions and the second prediction is determined to exceed a threshold value, request updated first local environment data and/or transmitting updated second local environment data to at least one of the plurality of nodes (200),
wherein the first prediction and the second prediction each describe a representation of the state of the tree and/or forest.

2. The cloud server (100) according to claim 1, wherein the first local environment data is related to at least one of a Biogenic Volatile Organic Compound, the body of a tree, temperature, humidity, pressure, light, and sound.

3. The cloud server (100) according to claim 1 or 2, wherein the second local environment data is related to at least one of satellite data, data from an aircraft, ground based sensor data, manual measurements, weather data, and soil data.

4. The cloud server (100) according to any one of claims 1 to 3, wherein the cloud server (100) is further configured to:
compare the second prediction of the local environment with the at least one of the first predictions of the local environment and the result thereof with at least one predetermined threshold value,
determine, based on a result of the comparison, which part of the first local environment data and which part of the second local environment data is required to sync the at least one of the first predictions of the local environment and the second predictions of the local environment with each other,
wherein further the requested updated first local environment data and/or the transmitted updated second local environment data corresponds to this determined required data.

5. The cloud server (100) according to claim 4, wherein comparing the second prediction of the local environment, the at least one of the first predictions of the local environment, and the at least one predetermined threshold value comprises:
a predetermined threshold value that indicates that first local environment data is required to sync the at least one of the first predictions of the local environment and the second predictions of the local environment with each other, or that second local environment data is required to sync the at least one of the first predictions of the local environment and the second predictions of the local environment with each other.

6. The cloud server (100) according to claim 4 or 5, wherein the determination which data requires updating is done such that the data transmitted from the cloud server (100) to the at least one node (200) is minimized.

7. The cloud server (100) according to claim 4 or 5, wherein the determination which data requires updating is done such that the data requested by the cloud server (100) from the at least one node (200) is minimized.

8. The cloud server (100) according to any one of claims 1 to 7, wherein requesting updated first local environment data comprises updating a local sensor protocol or a local sensor configuration.

9. The cloud server (100) according to any one of claims 1 to 8, wherein requesting updated first local environment data comprises updating a local model.

10. The cloud server (100) according to any one of claims 1 to 9, wherein requesting updated first local environment data comprises updating local parameters.

11. The cloud server (100) according to any one of claims 1 to 10, wherein the cloud server is further configured to determine an herbivore attack communication event on the basis of the second prediction of the local environment.

12. A node (200) for assessing tree and forest health via measurement of tree communication through air borne particles, the node being provided in the vicinity of a tree and being configured to:
sense first local environment data including data related to air borne particles;
receive second local environment data from a cloud server (100);
perform a first prediction of the local environment based on the first local environment data and the second local environment data using a local model using a Bayesian network;
transmit the first local environment data to the cloud server (100); and
transmit the first prediction of the local environment to the cloud server (100),
wherein the first prediction describes a representation of the state of the tree and/or forest.

13. A system for assessing tree and forest health via measurement of tree communication through air borne particles, the system comprising at least one node (200) according to claim 12 and a cloud server (100) according to any of claims 1 to 11.

14. A method for assessing tree and forest health by measurement of tree communication through air borne particles, the method comprising the steps of:
receiving first local environment data including data related to air borne particles from a plurality of nodes (200);
transmitting second local environment data to the plurality of nodes (200);
receiving at least one first prediction of the local environment from at least one of the plurality of nodes (200) based on the first local environment data using a local model using a Bayesian network;
performing a second prediction of the local environment based on the first local environment data and the second local environment data using a local model using a Bayesian network;
comparing the second prediction of the local environment with at least one of the first predictions of the local environment using a global model using a Bayesian network; and
when the difference between the at least one of the first predictions and the second prediction is determined to exceed a threshold, requesting updated first local environment data and/or transmitting updated second local environment data to at least one of the plurality of nodes (200),
wherein the first prediction and the second prediction each describe a representation of the state of the tree and/or forest.

15. A method for assessing tree and forest health via measurement of tree communication through air borne particles, the method comprising the steps of:
sensing first local environment data including data related to air borne particles;
receiving second local environment data from a cloud server (100);
performing a first prediction of the local environment based on the first local environment data and the second local environment data using a local model using a Bayesian network;
transmitting the first local environment data to the cloud server (100); and
transmitting the first prediction of the local environment to the cloud server (100),
wherein the first prediction and the second prediction each describe a representation of the state of the tree and/or forest.
